# EUROPEAN PATENT APPLICATION

(11) **EP 0 789 081 A2**
(43) Date of publication of application: **13.08.1997**
(21) Application number: 96901812.6
(22) Date of filing: 16.02.1996
(51) Int. Cl.: C12Q 1/68, C12Q 1/70

(54) **PROCESS FOR THE GENOME AMPLIFICATION AND MIXTURES OF INDUCER OLIGONUCLEOTIDES FOR THE DETECTION AND IDENTIFICATION OF RELATED INFECTIOUS AGENTS**

(30) Priority: 17.02.1995 ES 9500320
(71) Applicant: INSTITUTO DE SALUD CARLOS III, 28029 Madrid (ES)
(72) Inventor: CASAS, Inmaculada, 28220 Majadahonda (ES); TENORIO, Antonio, Centro Nac. de Microbiol., Virol, 28220 Majadahonda (ES); ECHEVARRIA, José Manuel, Centro Nac.de Microbiol.,, Majadahonda-Pozuelo, Km (ES); KLAPPER, Paul E., University of Manchester, Manchester (GB); CLEATOR, Graham M. University of Manchester, Manchester (GB)
(74) Representative: Maldonado Jordan, Julia
(86) International application number: ES9600031
(87) International publication number: WO9625909

(57) **Abstract**

In order to initiate the detection reaction, a combination of mixtures of inducer oligonucleotides is used, each of said mixtures being specifically designed for a family of infectious agents with genomically related sequences, and being obtained by simple addition of homologous oligonucleotides at its 3' extremity. The amplification reaction may be carried out in the same way while including an internal amplification control. The amplification products may be later discriminated by hybridation or by a second amplification reaction. The processes and mixtures of the invention are specially useful for the differential diagnosis of infectious agents which produce similar pathologies, specially the human herpes viruses and enteroviruses.

SEQUENCE LISTING
(1) GENERAL INFORMATION:
(i) APPLICANT:
(A) NAME: INSTITUTO DE SALUD CARLOS III
(B) STREET: Sinesio Delgado, 6 / Pabellón 3
(C) CITY: Madrid
(D) STATE: Madrid
(E) COUNTRY: Spain
(F) POSTAL CODE (ZIP): E-28029
(G) TELEPHONE: + 34 1 3230141
(H) TELEFAX: + 34 1 3231943
(ii) TITLE OF INVENTION: PROCEDIMIENTOS DE
AMPLIFICACION DE GENOMA Y MEZCLAS DE OLIGONUCLEOTIDOS
INICIADORES PARA LA DETECCION Y LA IDENTIFICACION DE
SECUENCIAS GENOMICAS RELACIONADAS
(iii) NUMBER OF SEQUENCES: 32
(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30
(EPO)
(2) INFORMATION FOR SEQ ID NO: 1:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: cDNA to mRNA
(iii) HYPOTHETICAL: YES
(iv) ANTI-SENSE: NO
(vi) ORIGINAL SOURCE:
(A) ORGANISM: Enterovirus
(xi) SEQUENCE DESCRIPTION: SEQ ID NO: 1:
CGGTACCTTT GTGCCCCTGT TTTA 24

(2) INFORMATION FOR SEQ ID NO: 2:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: cDNA to mRNA
(iii) HYPOTHETICAL: YES
(iv) ANTI-SENSE: NO
(vi) ORIGINAL SOURCE:
(A) ORGANISM: Enterovirus
(xi) SEQUENCE DESCRIPTION: SEQ ID NO: 2:
CGGTACCTTT GTACGCCTGT TTTA 24
(2) INFORMATION FOR SEQ ID NO: 3:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: cDNA to mRNA
(iii) HYPOTHETICAL: YES
(vi) ORIGINAL SOURCE:
(A) ORGANISM: Enterovirus
(xi) SEQUENCE DESCRIPTION: SEQ ID NO: 3:
GAAACACGGA CACCCAAAGT AGTCG 25
(2) INFORMATION FOR SEQ ID NO: 4:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:

(A) ORGANISM: Herpes simplex virus
(xi) SEQUENCE DESCRIPTION: SEQ ID NO: 4:
CGCATCATCT ACGGGGACAC GGA 23
(2) INFORMATION FOR SEQ ID NO: 5:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(vi) ORIGINAL SOURCE:
(A) ORGANISM: Varicella-zoster virus
(xi) SEQUENCE DESCRIPTION: SEQ ID NO: 5:
AAGGTTATAT ATGGAGATAC GGA 23
(2) INFORMATION FOR SEQ ID NO: 6:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Human cytomegalovirus
(xi) SEQUENCE DESCRIPTION: SEQ ID NO: 6:
CGGGTCATCT ACGGGGACAC GGA 23
(2) INFORMATION FOR SEQ ID NO: 7:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA (genomic)

(iii) HYPOTHETICAL: YES
(iv) ANTI-SENSE: NO
(vi) ORIGINAL SOURCE:
(A) ORGANISM: Human herpesvirus 6
(xi) SEQUENCE DESCRIPTION: SEQ ID NO: 7:
GAGGTAATTT ATGGTGATAC GGA 23
(2) INFORMATION FOR SEQ ID NO: 8:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Epstein-Barr virus
(xi) SEQUENCE DESCRIPTION: SEQ ID NO: 8:
CGAGTCATCT ACGGGGACAC GGA 23
(2) INFORMATION FOR SEQ ID NO: 9:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Herpes simplex virus
(xi) SEQUENCE DESCRIPTION: SEQ ID NO: 9:
ATGACGCCGA TGTACTTTTT CTT 23
(2) INFORMATION FOR SEQ ID NO: 10:
(i) SEQUENCE CHARACTERISTICS:
(A) LENGTH: 23 base pairs

(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Varicella-zoster virus
(xi) SEQUENCE DESCRIPTION: SEQ ID NO: 10:
ATTACCCCAA TGTACTTTTT CTT 23
(2) INFORMATION FOR SEQ ID NO: 11:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Human cytomegalovirus
(xi) SEQUENCE DESCRIPTION: SEQ ID NO: 11:
ACTTTACCAA TGTATCTTTT CTT 23
(2) INFORMATION FOR SEQ ID NO: 12:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Human herpesvirus 6
(xi) SEQUENCE DESCRIPTION: SEQ ID NO: 12:

TGTCTACCAA TGTATCTTTT TTT 23
(2) INFORMATION FOR SEQ ID NO: 13:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Epstein-Barr virus
(xi) SEQUENCE DESCRIPTION: SEQ ID NO: 13:
AGCACCCCCA CATATCTCTT CTT 23
(2) INFORMATION FOR SEQ ID NO: 14:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: cDNA to mRNA
(iii) HYPOTHETICAL: YES
(iv) ANTI-SENSE: NO
(vi) ORIGINAL SOURCE:
(A) ORGANISM: Enterovirus
(xi) SEQUENCE DESCRIPTION: SEQ ID NO: 14:
CAAGCACTTC TGTTTCCCCG G 21
(2) INFORMATION FOR SEQ ID NO: 15:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: cDNA to mRNA
(iii) HYPOTHETICAL: YES

(iv) ANTI-SENSE: NO
(vi) ORIGINAL SOURCE:
(A) ORGANISM: Enterovirus
(xi) SEQUENCE DESCRIPTION: SEQ ID NO: 15:
TAGCTCAATA GGCTCTTCAC ACC 23
(2) INFORMATION FOR SEQ ID NO: 16:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Herpes simplex virus
(xi) SEQUENCE DESCRIPTION: SEQ ID NO: 16:
GTGTTGTGCC GCGGTCGCAC 20
(2) INFORMATION FOR SEQ ID NO: 17:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Varicella-zoster virus
(xi) SEQUENCE DESCRIPTION: SEQ ID NO: 17:
TGAGGGGATA GCTAAAATCC 20
(2) INFORMATION FOR SEQ ID NO: 18:
(i) SEQUENCE CHARACTERISTICS:
(A) LENGTH: 20 base pairs
(B) TYPE: nucleic acid

(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Human cytomegalovirus
(xi) SEQUENCE DESCRIPTION: SEQ ID NO: 18:
GGGCCCAGCC TGGCGCACTA 20
(2) INFORMATION FOR SEQ ID NO: 19:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Human herpesvirus 6
(xi) SEQUENCE DESCRIPTION: SEQ ID NO: 19:
GCCAAACATA TCACAGATCG 20
(2) INFORMATION FOR SEQ ID NO: 20:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Epstein-Barr virus
(xi) SEQUENCE DESCRIPTION: SEQ ID NO: 20:
ACCCGGAGCC TGTTTGTAGC 20

(2) INFORMATION FOR SEQ ID NO: 21:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Herpes simplex virus
(xi) SEQUENCE DESCRIPTION: SEQ ID NO: 21:
GGTGAACGTC TTTTCGAACT C 21
(2) INFORMATION FOR SEQ ID NO: 22:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Varicella-zoster virus
(xi) SEQUENCE DESCRIPTION: SEQ ID NO: 22:
TATAAAAGTT TTTTCACACT C 21
(2) INFORMATION FOR SEQ ID NO: 23:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO

(vi) ORIGINAL SOURCE:
(A) ORGANISM: Human cytomegalovirus
(xi) SEQUENCE DESCRIPTION: SEQ ID NO: 23:
GACGAAGACC TTTTCAAACT C 21
(2) INFORMATION FOR SEQ ID NO: 24:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Human herpesvirus 6
(xi) SEQUENCE DESCRIPTION: SEQ ID NO: 24:
ACATAAAATC TTTTCAAACT C 21
(2) INFORMATION FOR SEQ ID NO: 25:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Epstein-Barr virus
(xi) SEQUENCE DESCRIPTION: SEQ ID NO: 25:
GGAGAAGGTC TTCTCGGCCT C 21
(2) INFORMATION FOR SEQ ID NO: 26:
(i) SEQUENCE CHARACTERISTICS:
(A) LENGTH: 25 base pairs
(B) TYPE: nucleic acid
(C) STRANDEDNESS: single

(D) TOPOLOGY: linear
(ii) MOLECULE TYPE: cDNA to mRNA
(iii) HYPOTHETICAL: YES
(iv) ANTI-SENSE: NO
(vi) ORIGINAL SOURCE:
(A) ORGANISM: Enterovirus
(xi) SEQUENCE DESCRIPTION: SEQ ID NO: 26:
CCTCCGGCCC CTGAATGCGG CTAAT 25
(2) INFORMATION FOR SEQ ID NO: 27:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Pseudorabies virus
(xi) SEQUENCE DESCRIPTION: SEQ ID NO: 27:
CGCGTGGTCT ACGGGGACAC GGA 23
(2) INFORMATION FOR SEQ ID NO: 28:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Pseudorabies virus
(xi) SEQUENCE DESCRIPTION: SEQ ID NO: 28:
ATGACGCCGA TGTATTTCTT CTT 23
(2) INFORMATION FOR SEQ ID NO: 29:

(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Pseudorabies virus
(xi) SEQUENCE DESCRIPTION: SEQ ID NO: 29:
GGGACACGGA CTCGGTCTTC 20
(2) INFORMATION FOR SEQ ID NO: 30:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Pseudorabies virus
(xi) SEQUENCE DESCRIPTION: SEQ ID NO: 30:
CCGGAAGGTC TTCTCGCACT C 21
(2) INFORMATION FOR SEQ ID NO: 31:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:

(A) ORGANISM: Herpes simplex virus type 1
(xi) SEQUENCE DESCRIPTION: SEQ ID NO: 31:
GGGCTCACGG CCGCCGGGCT GACG 24
(2) INFORMATION FOR SEQ ID NO: 32:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
(A) ORGANISM: Herpes simplex virus type 2
(xi) SEQUENCE DESCRIPTION: SEQ ID NO: 32:
GACACGGACT CCATTTTCGT TT 22

## Description

### Field of the Invention

The invention relates to new mixtures of reaction primers and to new genomic amplification processes useful for the detection and specific identification of related infectious agents, especially infectious agents capable of producing neurologic disease, and more specifically for the detection and specific identification of human herpesviruses and enteroviruses.

### State of the Art

One of the work objectives of a diagnosis laboratory, which is often faced with the need of establishing a differential diagnosis, as it occurs, among others, in the diagnosis of acute neurologic infection, is the detection and characterization of the different infectious agents. Neurologic infections and, more specifically, those of viral origin, are produced, among others, by viruses belonging to two taxonomically unrelated Families: *Herpesviridae* and *Picornaviridae*. In the first place, herpesviruses are infectious agents which after an initial acute infection establish a latent infection, producing, under specific conditions, periodic recurrences. They are enveloped viruses whose genome is constituted by a double-stranded DNA molecule. Essentially, neurologic pathology is associated to the recurrence of Herpes simplex virus type 1 (HSV1), Herpes simplex virus type 2 (HSV2) and Varicella-zoster virus (VZV). Cytomegalovirus (CMV), Human herpesvirus 6 (HHV6) and Epstein-Barr virus (EBV) are also possible agents directly related with certain syndromes which lead to a neurologic alteration. The most common clinical manifestations of the infections of the central nervous system associated with this group of viruses are aseptic meningitis, encephalitis, meningoencephalitis and polyradiculitis. In the second place, the latter, included within the Picornaviridae family, comprises the enterovirus group, a genus known since the XIVth century B.C. for the production of poliomyelitis epidemics. Infection by these small-sized unenveloped viruses, the genome of which is constituted by a single-stranded RNA molecule of positive polarity, has a greater incidence during the first years of life, generating a wide range of clinical syndromes. In developed countries, they are the most frequent etiologic agents in cases of aseptic meningitis, a neurologic syndrome which, in Spain, is associated with enteroviruses in 90% of total cases, depending on the year and the total number of epidemic outbreaks which have taken place.

Both enteroviruses and herpesviruses are capable of producing neurological syndromes, both in immunocompetent as well as in immunodepressed patients. Due to its high frequency, aseptic meningitis, in addition to encephalitis and meningoencephalitis, because of its less favorable clinical evolution, are the subject of different studies directed towards the discovery of the viruses involved in their production.

The control of acute neurological diseases can be undertaken by the establishment of an effective diagnosis which allows the discovery and the identification of the etiologic agent associated with a specific combination of neurological symptoms. The detection and identification of these infectious agents is one of the basic needs of the microbiological diagnosis laboratory. Currently, laboratory diagnosis is based upon the isolation of the different viruses in cultured cells. This technique is slow and, furthermore, requires an additional effort to maintain different cell lines with the object of being able to cover as many enterovirus serotypes as possible, apart from the different herpesviruses. A positive result in isolating can be established in a period of time between 4 and 15 days. However, certain enteroviruses are incapable of growing in cell cultures, thus making their detection impossible in this type of diagnosis systems.

In relation to serological techniques, indirect systems for the analysis of specific antibodies produced against certain herpesviruses are extremely useful when the analyses are carried out on samples where the antibody titer is high. This situation is different in those cases of neurologic infection where the sample to be analyzed is, essentially, cerebrospinal fluid (CSF). In this sort of samples the specific antibody titer against a virus can be very low and barely detectable. On the other hand, indirect methods of analysis for the detection of specific antibodies against enteroviruses have no diagnostic value, due to their free circulation as normal pathogens within a healthy population. Additionally, the high antigenic diversity of the enteroviruses does not permit the development of serological techniques which are sufficiently sensitive and specific to identify all its types.

The introduction of genomic amplification techniques (PCR) for viral infectious agents in clinical samples has meant one of the greatest advances in the detection of the etiologic agent of numerous infectious diseases, and hence also in the establishment of a differential etiologic diagnosis. Thus, processes for the genomic amplification of herpesviruses and enteroviruses in clinical samples, are widely documented in the literature. Specifically, in the RNA amplification process, it is necessary to carry out a first initial phase in which a specific complementary DNA molecule must be produced by means of a process of reverse transcription. Of particular importance are those protocols in which the combined amplification of different viruses in a single reaction (multiplex-PCR) have been designed, as is the case of herpesviruses (Spanish Patent P9201174). Viruses which belong to a same family, and which are intrinsically related with one another, exhibit a high degree of homology between certain genomic regions; for instance, the 5' non-coding region, in the case of enteroviruses, and the region which encodes the DNA polymerase gene, in the case of herpesviruses. These highly conserved genomic regions are the subject of molecular studies, and common and specific primers are designed based upon them, which will be employed in PCR techniques to detect and identify each of the viruses in a single assay. The main advantage of this type of assays is the saving of clinical sample and time involved in obtaining a differential etiologic diagnosis.

The use of multiple amplification techniques does not only allow the detection of any enterovirus or herpesvirus, including those which are difficult to culture, but also permits, in the case of herpesviruses, their specific identification, even if they are present in the minute quantities found in CSF. It must be remarked that multiple amplification techniques also present a number of drawbacks and problems which are difficult to solve. The problems posed by non-specificity, reproducibility and lack of sensitivity are, among others, matters which, *a priori*, have to be considered, especially at the time of designing the specific primers for each of the viruses which are to be amplified. Once the primers have been designed, the standarisation phase of the multipie amplification technique is essential, and it is during this period when the greatest incompatibility problems arise at all levels. The interactions occurring between specific primers of the corresponding viruses to be detected and the human genome, which is a molecule which exhibits a high degree of complexity, generate undesirable non specific amplifications. It must be taken into account that the human DNA molecule is present in every type of clinical sample in
1. using a combination of oligonucleotide primer mixtures, each mixture being specifically designed for a family of infectious agents with genomically related sequences, and itself characterized in that :
   i) it is obtained by the simple addition of oligonucleotides,
   ii) each of the oligonucleotides comprised in said mixture include, preferably at their 3' end, homologous sequences selected from among the related sequences which are desired to amplify,
   iii) each of the oligonucleotides comprised in said mixture may further include, preferably at their 5' end, non-homologous sequences selected from among the related sequences which are desired to amplify, and
   iv) one or more of the oligonucleotides comprised in said mixture may be differentiated from the known sequences which are desired to amplify in at least one nucleotide, and
2. combining all the mixtures designed according to the previous features in a single mixture which will be used in the genomic amplification reaction.

The term "homologous sequences", in the sense it is used in this specification, refers to nucleotide sequences which have a degree of homology with each other greater than 50%, preferably greater than 70% and, even more preferably, greater than 90%.

Another aspect of the invention are mixtures such as those described for the previous process.

The use of the processes and mixtures previously described provides, in addition to the advantages achieved by the individual use of the mixtures described which essentially consist in an improvement in the specificity and sensitivity of the reaction, the added advantage of being able to detect, in a single amplification reaction, which the specific detection of the viral agents involved in a specific infectious disease is carried out. Additionally, the presence of radically different agents, such as herpesviruses and enteroviruses, in a same sample, may involve a system failure in relation to the reproducibility of the results obtained. Another factor to be considered is the initial step of reverse transcription of the enteroviral RNA molecules, as this can mean a loss in the availability of herpesvirus DNA molecules for amplification in the second step of polymerization (PCR).

False negatives are other of the problems which can be found when employing PCR techniques in the diagnosis laboratory, this is why a number of specific controls must be included, so as to exclude false negatives caused by the presence of inhibitors of the amplification reaction. Hence, the development and use of internal amplification controls in each reaction tube is very advisable.

The application of PCR technology, and the possible combinations of primers, to amplify genomic sequences of viruses associated with the production of neurologic infection, would cover one of the needs which arise in laboratory diagnosis.

### Description of the Invention

The present invention has the object of describing new mixtures of reaction primers and new genomic amplification processes of use for the detection and specific identification of related infectious agents, especially infectious agents capable of producing neurologic disease, and more specifically for the detection and specific identification of human herpesviruses and enteroviruses, which are viruses which have no significant homology at the genomic level.

An aspect of the invention is a genome amplification process for the detection of related infectious agents, in a single reaction mixture, characterized by infectious agents which produce similar pathologies but which are not genomically related among themselves, as is the case of viruses belonging to the *Herpesviridae* and *Picornaviridae* families. For each of the families, the improvement in sensitivity is achieved by reducing to a maximum the degree of degeneration of the primers, designed upon a region with relatively conserved sequences among the different members of the family. The improvement in the specificity is achieved by increasing, if it were necessary, the length of the primers, extending them at their 5' end towards regions which are not conserved among the different members of the family, thus achieving a primer length which is sufficient to avoid non-specific hybridization with similar sequences which may be present in highly complex genomes, such as the human genome.

The mixtures and processes previously described are of use in the detection of infectious agents which produce similar pathologies, but which are not genomically related to each other, especially those which produce pathologies in humans, as is the case of those belonging to the *Herpesviridae* and *Picornaviridae* families.

Another aspect of the invention is a process for the detection and the identification of genomically related sequences, characterized by:
i) carrying out a first genome amplification reaction in which at least one of the primers employed is in a mixture such as the one used in the first process described
ii) using the product of the first reaction as a substrate, the specific identification is carried out by means of hybridization with oligonucleotides which are specific to each of the species which one wishes to identify.

This process allows undertaking both the detection, as well as the identification, of related infectious agents, in two consecutive reactions, the first being one of genomic amplification and the second of specific hybridization, thus making possible both the detection, as well as the identification, of related infectious agents.

Another aspect of the invention is a genome amplification process for the detection and the identification of related infectious agents in a single reaction mixture, characterized in that at least one of the primers employed is a mixture characterized in that
i) it is obtained by the simple addition of oligonucleotides,
ii) each of the oligonucleotides comprised in said mixture include, preferably at their 3' end, specific sequences from each one of the sequences which one wishes to identify in the amplification reaction,
iii) it is designed in such a manner that the amplified specific fragments differ from each other by reason of size or physical or chemical labelling, from each of the oligonucleotides comprised in the mixture, and
iv) one or more of the oligonucleotides comprised in said mixture may be differentiated from the known sequences which one wishes to amplify in at least one nucleotide.

Another aspect of the invention are mixtures such as those described for the previous process.

Another aspect of the invention is a process for the detection and the identification of genomically related sequences, characterized in that:
i) a first genome amplification reaction is carried out in which at least one of the primers employed is in a mixture such as the one used in the first process described
ii) using the product of the first reaction as a substrate, a second amplification reaction is carried out in which a mixture is used such as the one used in the previously described process.

This process allows, equally, undertaking both the detection, as well as the identification, of related infectious agents, in two consecutive reactions.

Also, according to the invention, the complex mixture of primers used in the second reaction does not produce the non-specific amplifications caused by their use in the presence of a complex genome. This is achieved by using the reaction product obtained from the first amplification reaction as substrate for the second reaction.

The mixtures and processes previously described are especially useful in the detection and specific identification of different infectious agents which are related because they produce a similar pathology, especially in man.

Another aspect of the invention is the inclusion of an internal amplification control within the processes and mixtures previously described, which are used for the detection and identification of related infectious agents. Thus, according to the invention, the previous processes and mixtures may include, within the reaction mixture itself, specific amounts of a genome which is not expected to be found in a clinical sample, in addition to the reaction primers and other specific reagents which allow its detection in the event the amplification reaction has taken place in the absence of inhibitors. The inclusion of an internal amplification control will allow the detection of the samples which contain amplification inhibitors, hence easily identifying false negative results.

A last aspect of the invention are oligonucleotide mixtures designed to be used as primers of the amplification reaction, characterized in that at least one of the oligonucleotide components of said mixture differs from the known sequences which one wishes to amplify in at least one nucleotide.

The alteration of the sequence according to the invention, has the main objective of achieving that the complex mixtures of primers will be compatible with each other; that is, that no hybridizations will take place between the different components of the oligonucleotide mixtures present in the amplification reaction mixture. Hybridization between oligonucleotides, which are frequent in complex mixtures, leads to the scavenging of the necessary reagent during the amplification reaction and, frequently, to the formation of primer dimers.

An embodiment of the invention consists in the application of the processes and mixtures previously described, for the detection and identification of infectious agents which are related because they are capable to produce a similar pathology, especially in man, especially those capable of producing neurologic disease in man, particularly those belonging to the *Herpesviridae* and *Picornaviridae* families.

The following examples and figures are given to illustrate, but not to limit, some of the modes of carrying out the invention.

### Description of the Figures

### Figure 1

Alignment of the nucleotide sequences corresponding to the 5' non-coding regions (5'NCR) of the enteroviruses of known sequence. The names of the viruses and their origin are described next, as well as the sequence recognition code in the database from which they have been obtained (GenBank), in square brackets, and their identification in the figure, in brackets. Poliovirus type 1, strain Mahoney [POL1] (POL1A); Poliovirus type 1, Mahoney strain version A [POLIO1A] (POL1B); Poliovirus type 1, Mahoney strain version B [POLIO1B] (POL1C); Poliovirus type 1, Sabin strain 1 [POLIOS] (POL1D); Poliovirus type 2, Sabin strain 2 [PIPOLS2] (POL2A); Poliovirus type 2 [POL2CG1] (POL2B); Poliovirus type 2, Lansing strain [POL2LAN] (POL2C); Poliovirus type 3, Sabin strain 3 [PIPOL3] (POL3A); Poliovirus type 3, strain 23127 [PIPO3XX] (POL3B); Poliovirus type 3, Leon 37 strain [POL3L37] (POL3E); Poliovirus type 3, strain P3/119 [PIPO3119] (POL3C); Poliovirus type 3, strain Leon 12a1b [POL3L12C] (POL3D); Coxackievirus A21 [CXA21CG] (CA21); Coxackievirus B1 [CXA1g] (CB1); Coxackievirus B3 [CXA3CG] (CB3); Coxackievirus B4 [PICOXB4] (CB4); Coxackievirus B4 [CXB4S] (CB4S); Coxackievirus B5 [CXB5CGA] (CB5); Coxackievirus A16 [CAU05876] (CA16); Coxackievirus A9 [CXA9CG] (CA9); Coxackievirus A24 [CXA24CG] (CA24); Echovirus 12 [EC12TCG] (E12); Echovirus 12, wild type Travis prototype [EC12TCGW] (E12W). The corresponding sequences of the enterovirus primers are identified with the sequence code (SEQ ID No:). The dashes represent the absence of the corresponding nucleotide in the alignments.

### Figure 2

Schematic representation of the nucleotide sequence alignments corresponding to: (A) target regions of the DNA polymerase genes of the 6 human herpesviruses: Herpes simplex type 1 (HSV1); Herpes simplex type 2 (HSV2); Varicella-zoster virus (VZV); Cytomegalovirus (HCMV); Human herpesvirus 6 type A (HHV6-A); Epstein-Barr virus (EBV); of human DNA polymerase (HUMAN) and of the Porcine pseudorabies virus (PRV), (B) Oligonucleotide primers of the first amplification reaction and (C) Oligonucleotide primers of the second amplification reaction. Each oligonucleotide is identified with the sequence code (SEQ ID No:).

### Figure 3

Agarose gel electrophoresis of the amplification fragments generated in Example 2. Molecular weight marker (M); Coxsackievirus A 16 (lane 1); HSV1 (lane 2); HSV2 (lane 3); VZV (lane 4); CMV (lane 5); HHV6 (lane 6); EBV (lane 7); Mixture of all the herpesviruses (lane 8); Human foetal fibroblasts (lanes 9 and 17); Coxsackievirus A16 and HSV1 (lane 10); Coxsackievirus A16 and HSV2 (lane 11); Coxsackievirus A16 and VZV (lane 12); Coxsackievirus A16 and CMV (lane 13); Coxsackievirus A16 and HHV6 (lane 14); Coxsackievirus A16 and EBV (lane 15); Coxsackievirus A16 and a mixture of all the herpesviruses (lane 16). The 500 bp fragment corresponds to the enteroviruses and the 194 bp to the herpesviruses.

### Figure 4

Agarose gel electrophoresis of the amplification fragments generated in Example 4. Molecular weight marker (M); molecular weight marker of the amplified fragments: 120 bp HSV1 and HSV2, 98 bp VZV, 78 bp CMV, 66 bp HHV6 and 54 bp EBV (lanes 1, 10 and 19). HSV1, HSV2, VZV, CMV, HHV6, EBV (lanes 2, 3, 4, 5, 6 and 7 without Coxsackievirus A16 RNA respectively, lanes 11, 12, 13, 14, 15 and 16 in the presence of Coxsackievirus A16 RNA respectively). Mixture of all the herpesviruses (lanes 8 and 17 with and without Coxsackievirus A16 RNA respectively); Human fibroblasts (lanes 9 and 18).

### Figure 5

Agarose gel electrophoresis of the amplification fragments generated in the first and second amplifications according to Example 6. Molecular weight marker (M), 500 bp fragment corresponds to the amplification of Coxsackievirus A16 (lanes 1, 3 and 4), the 194 bp fragment corresponds to the first amplification of PRV (lanes 2, 3 and 4). Herpesvirus molecular weight marker (lane 6), Coxsackievirus A16 in second amplification (lane 7), identification of PRV without enterovirus (lane 8), with enterovirus (lane 9), in a mixture of all the herpesvirus (lane 10), Human foetal fibroblasts (lanes 5 and 11).

### Figure 6

Agarose gel electrophoresis of the amplification fragments generated in the second amplification of herpesviruses, with or without internal amplification control, according to Example 7. Molecular weight marker (M); mixture of all the herpesviruses HSV1, HSV2, VZV, CMV, HHV6, EBV and Human foetal fibroblasts with second herpesvirus multiple amplification reaction primers (lanes 1 to 8 respectively), with PRV primers apart from those for herpesvirus multiple amplification (lanes 9 to 16 respectively), with PRV and PRV DNA primers (lanes 17 to 24 respectively).

### Figure 7

1) Agarose gel electrophoresis of the amplification fragments generated in the first and second amplifications according to Example 10. Molecular weight marker (M), 500 bp fragment for Coxsackievirus A16 (lane 1) and the 194 bp fragments for HSV1 (lanes 2 and 3) and HSV2 (lane 4). After typification, specific amplification of HSV1 (lane 5) and HSV2 (lane 6) is observed.
2) Agarose gel electrophoresis of four clinical samples in which HSV1 (lanes 1 and 2) and HSV2 (lanes 3 and 4) had been identified prior to genomic amplification. Human foetal fibroblasts (lanes 5 and 6). Molecular weight marker (M).

In a practical embodiment of this invention, a method is provided for the differential diagnosis of infectious agents which are related by their ability to produce a similar pathology, belonging to different families which are not genomically related to each other, which includes (a) the detection of said infectious agents by enzymatic amplification of a fragment of their genomes and (b) the identification of said infectious agents starting from the products of the amplification reaction, wherein said amplification reaction is carried out in a single amplification mixture which comprises, as primer for the reaction, a combination of oligonucleotide mixtures, each one of which has been specifically designed for each one of the families of infectious agents to be detected, so that each mixture of oligonucleotides:
i) is obtained by the simple addition of oligonucleotides,
ii) each one of the oligonucleotides comprised in said mixture include, preferably at their 3' end, homologous sequences selected from among the genomically related sequences which are desired to amplify,
iii) each one of the oligonucleotides comprised in said mixture may further include, preferably at their 5' end, non-homologous sequences selected from among the genomically related sequences which are desired to amplify, and
iv) the oligonucleotide components of which may be differentiated from the known sequences which are desired to amplify in at least one nucleotide.

A specific application of said method consists in the detection and identification of infectious agents which produce a similar pathology in humans, particularly, a neurologic pathology, such as the one caused by infectious agents belonging to the *Herpesviridae* end *Picornaviridae* families, particularly that caused by human herpesviruses and enteroviruses.

In an alternative embodiment of said method, the amplification reaction mixture also includes an internal amplification control, which comprises the genome of a virus which must not be present in the sample to be studied, together with reaction primers specific to a fragment of said genome. Said genome is preferably derived from a viral species belonging to one of the viral families to be identified, and which is incapable of infecting the animal species, for example the human one, on which the diagnosis is carried out. In a particular embodiment, said genome corresponds to a herpesvirus which cannot infect man, such as Porcine herpesvirus 1.

Once detected, the identification of the infectious agent can be performed either by hybridization of the amplified sequences with specific oligonucleotide probes or specific oligonucleotide mixtures for the species or genera to be identified or, alternatively, by means of a second amplification reaction over the products resulting from the first amplification reaction, in which the reaction mixture for said second amplification reaction comprises, as primer for the reaction, a mixture of oligonucleotides designed in such a way that:
i) it is obtained by the simple addition of oligonucleotides,
ii) each one of the oligonucleotides comprised in said mixture include, preferably at their 3' end, specific sequences of each of the sequences to be typified in said second amplification reaction,
iii) the specific amplified fragments differ from each other by reason of size or different physical or chemical labelling of each one of the oligonucleotides comprised in said mixture, and
iv) one or more of the oligonucleotides comprised in said mixture may be differentiated from the known sequences which one wishes to amplify in at least one nucleotide.

With the object of confirming the diagnosis, the method proposed also envisages the possibility of confirming the identity of each viral species identified by means of an additional specific hybridization reaction with an oligonucleotide which is specific of each species the identity of which one wishes to confirm, or alternatively, by an additional genomic amplification reaction using the appropriate oligonucleotide primers. In this way, the diagnosis method provided by this invention permits the specific identification of herpes simplex virus (type 1 or type 2) in a sample (see Example 10).

### Examples

### Example 1. Design of primers for the detection of human enteroviruses with the object of obtaining an enterovirus and/or herpesvirus multiple amplification method.

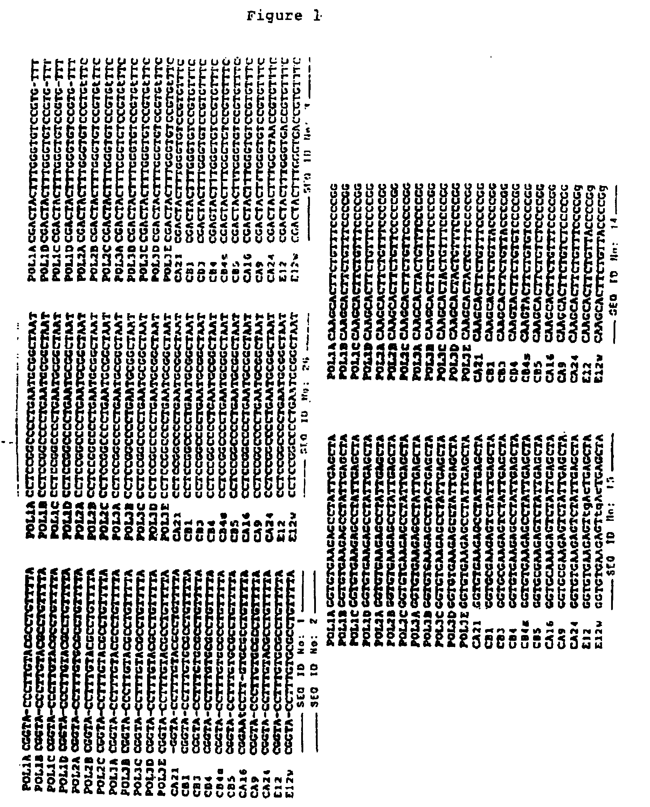

The genus enterovirus comprises a very large number of viruses, having it only been possible to obtain the complete sequence of their genome in a minority of cases. The 5' non-coding region (5'NCR) stands out remarkably due to the enormous homology exhibited among the enteroviruses sequenced to date, the sequences of which are available from the GENBANK database. The 5'NCR region is of great biological interest due to its involvement in the beginning of the replication of this group of viruses. Exceptionally, enterovirus 22 and Coxsackievirus A2, which do not exhibit homology in this region of the genome, have been excluded from the conserved gene block of the 5'NCR region of the available enteroviruses, which is represented in Figure 1.

On the basis of the comparative analyses of each one of the genomes studied, the following primers have been designed:
- POSITIVE POLARITY:: SEQ ID No: 1
SEQ ID No: 2
- NEGATIVE POLARITY:: SEQ ID No: 3

As it can be observed in Figure 1, the degree of homology between the two primers designed and the corresponding specific sequence of each one of the enteroviruses whose genome has been sequenced, is very high, even being exactly the same for most of the viruses. This high degree or homology allows the performance of very specific and highly sensitive enterovirus amplification reactions.

Once the enterovirus primers have been designed, the system must be made compatible so as to be able to amplify, in a first amplification reaction, RNA of enteroviral origin, as well as DNA of herpesvirus origin. To do this, a reaction mixture has been designed which comprises the primers for the enterovirus and herpesvirus first amplification reaction. The herpesvirus primers were designed and described in the Spanish Patent P9201174.

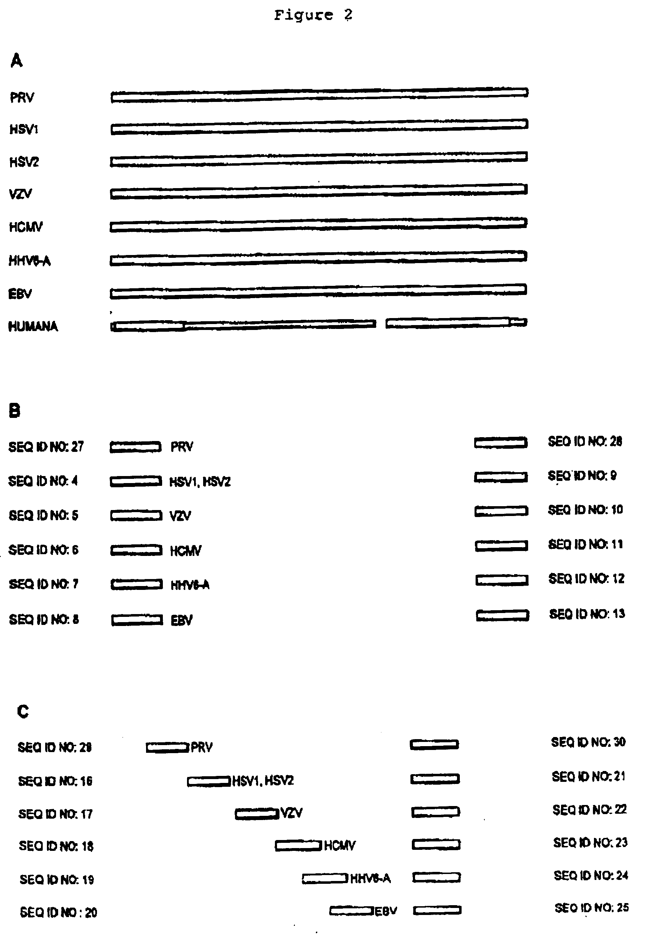

On the basis of the sequences depicted in Figures 1 and 2 and in accordance with Example 2 and with the processes described in the Spanish Patent P9201174, a mixture has been designed for the amplification of enteroviruses and herpesviruses in a single amplification reaction. The mixture of primers is comprised by:

| POSITIVE POLARITY | NEGATIVE POLARITY |
|---|---|
| SEQ ID No: 1 | SEQ ID NO: 3 |
| SEQ ID No: 2 | SEQ ID No: 9 |
| SEQ ID No: 4 | SEQ ID No: 10 |
| SEQ ID No: 5 | SEQ ID No: 11 |
| SEQ ID No: 6 | SEQ ID No: 12 |
| SEQ ID No: 7 | SEQ ID No: 13 |
| SEQ ID No: 8 | |

### Example 2. Detection of human enteroviruses and/or herpesviruses in a first amplification reaction.

To carry out a first amplification assay, the samples to be analyzed contain different dilutions of enteroviruses mixed with a specific amount of human fibroblasts. The method for preparing the viral RNA has been described previously (Casas I., Powell L., Klapper P.E., Cleator G.M. "A new extraction method for RNA and DNA by precipitation in clinical samples". J. Virol. Methods 1994, in press). Once the nucleic acids (DNA and RNA) present in the sample to be analyzed have been precipitated , they are diluted in 10 µL of water. The RNA present in 5 µL of the previous dilution is converted into cDNA by means of a reverse transcription, of 30 minutes duration at a temperature of 37 °C, under the conditions recommended by the manufacturer of the reverse transcriptase, an essential enzyme for this process. 100 pmol of negative polarity primer were used, as the RNA strand exhibits a positive polarity.

The cDNA will be subjected to 40 cycles of amplification, at a primer hybridization temperature of 60°C. The denaturation temperature range is fixed between 92°C and 95°C, although the temperature of choice is preferably 94°C. The DNA polymerase extension temperature is fixed between 60°C and 80°C, although it is preferably of 72°C. The reaction mixture is constituted by a mixture of the two primer mixtures at a chosen concentration of 100 pmol of each. The total reaction volume chosen is of 50 µL.

Samples containing 30 different types of enterovirus originating from isolates obtained in our laboratory were assayed using the primers designed in Example 1:
- Polio 1, wild type y vaccinial: Echovirus 7
- Polio 2, wild type y vaccinial: Echovirus 9
- Polio 3, wild type y vaccinial: Echovirus 11
- Coxackievirus B1: Echovirus 14
- Coxackievirus B2: Echovirus 17
- Coxackievirus B4: Echovirus 19
- Coxackievirus B5: Echovirus 20
- Coxackievirus B6: Echovirus 21
- Coxackievirus A9: Echovirus 24
- Coxackievirus A16: Echovirus 25
- Echovirus 2: Echovirus 27
- Echovirus 3: Echovirus 30
- Echovirus 4: Echovirus 31
- Echovirus 5: Echovirus 33
- Echovirus 6: Enterovirus 70

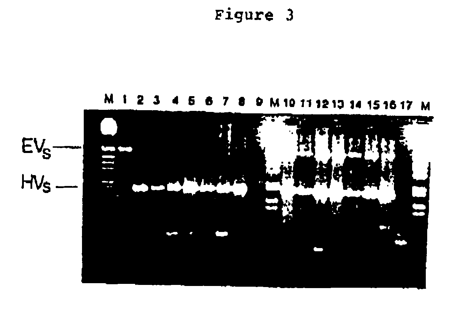

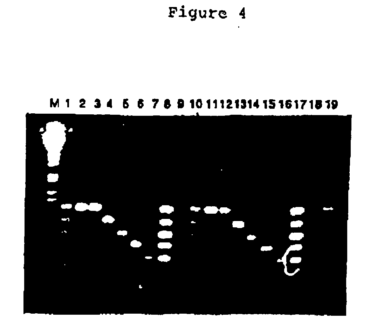

Samples containing 100 TCID₅₀ of Coxsackievirus A16 with HSV1, HSV2, VZV, CMV, HHV6 and EBV were assayed using the primers designed in Example 1. The analysis of the fragments generated is shown in Figure 3. All the enteroviruses generate amplification fragments of between 500 and 487 bp, and herpesviruses of 194 bp.

### Example 3. Design of primers for the specific detection of enteroviruses and/or herpesviruses in which the label is the molecular weight of the amplified fragment.

Primers for a second amplification reaction which uses the fragment obtained in the first amplification reaction as target molecule, have been designed for the identification of the fragments generated in Example 2. In this way, the sensitivity and specificity of the identification reaction are increased. The following primers, common to all the enteroviruses, have been initially selected on the basis of the sequences listed and compared in Figure 1:
- POSITIVE POLARITY:: SEQ ID No: 14
- NEGATIVE POLARITY:: SEQ ID No: 15

After the second amplification reaction, 272 bp amplification fragments are generated.

Based upon the comparison of the sequences of Figures 1 and 2 and according to the processes described in the Spanish Patent P9201174, a mixture has been designed for a second amplification which will employ the enterovirus and herpesvirus amplification fragments obtained in Example 2 as substrate. Said mixture is comprised by:

| POSITIVE POLARITY | NEGATIVE POLARITY |
|---|---|
| SEQ ID No: 14 | SEQ ID No: 15 |
| SEQ ID No: 16 | SEQ ID No: 21 |
| SEQ ID No: 17 | SEQ ID No: 22 |
| SEQ ID No: 18 | SEQ ID No: 23 |
| SEQ ID No: 19 | SEQ ID No: 24 |
| SEQ ID No: 20 | SEQ ID No: 25 |

### Example 4. Detection and identification by molecular weight of the amplified fragment of human herpesviruses in the presence of enterovirus.

1 : 1000 dilutions of the 194 bp fragments generated in the first amplification reaction of Example 2 were reamplified, using the mixtures of primers defined for a second herpesvirus amplification reaction, designed in the Spanish Patent P9201174, under the conditions described in said patent. As can be appreciated in Figure 4, it is possible to determine and identify the herpesvirus present in the sample, without this amplification being affected by the presence of the amplification fragments coming from enteroviral RNA. All human herpesviruses generated an amplification fragment of the molecular weight expected.

### Example 5. Design of a probe which is common and specific for the identification of human enteroviruses.

A specific probe has been designed, on the basis of the sequences listed and compared in Figure 1, for the identification of the fragments generated in Example 2. The region selected within the amplification fragment exhibits a high degree of homology, being virtually the same in all the enteroviruses compared. The nucleotide sequence is as follows:
- POSITIVE POLARITY:: SEQ ID No: 26

### Example 6. Design of the primers for the detection of an internal amplification control in each reaction tube.

The herpesvirus multiple amplification method previously described in the Spanish Patent P9201174, has been used as the base for the design. Said design is based on the multiple amplification of the herpesviruses HSV1, HSV2, VZV, CMV, HHV6, and EBV using a mixture of primers characterized in said patent in that their 3' ends are regions conserved among the DNA polymerase genes of human herpesviruses.

A cloned fragment of the DNA polymerase gene of the porcine herpesvirus PRV (Porcine pseudorabies virus or Auzjevski's disease) has been designed to be used as an internal amplification control. This DNA fragment which is added systematically to all the tubes, will be amplified by specific primers which have bean designed in the same manner as those for the rest of the herpesviruses, in the previously mentioned patent. Figure 2 shows the nucleotide sequences of the selected DNA polymerase fragments and the positive and negative polarity mixtures of the herpesviruses.

The sequences of the specific primers of the internal control or of the cloned fragment of the PRV virus are the following:
- POSITIVE POLARITY:: SEQ ID No: 27
- NEGATIVE POLARITY:: SEQ ID No: 28

Specific primers have been designed which are capable of reamplifying the fragment generated in the first PRV amplification reaction as internal control of amplification. The following primers have been designed for the second amplification reaction:
- POSITIVE POLARITY:: SEQ ID No: 29
- NEGATIVE POLARITY:: SEQ ID No: 30

The specific PRV fragment would have a size of 140 bp after the second amplification and hence, it is easily detectable.

### Example 7. Detection of herpesviruses in the presence of specific primers of the PRV virus used as internal control of the first and second amplification.

According to the process described in Example 4, a new mixture of specific herpesvirus primers has been employed, which also contains the specific PRV primers, used as internal amplification control. Initially, DNA from each of the herpesviruses has been subjected to a first amplification reaction, according to the processes described in Example 1, using a mixture of primers constituted by:

| POSITIVE POLARITY | NEGATIVE POLARITY |
|---|---|
| SEQ ID No: 27 | SEQ ID No: 28 |
| SEQ ID No: 4 | SEQ ID No: 9 |
| SEQ ID No: 5 | SEQ ID No: 10 |
| SEQ ID No: 6 | SEQ ID No: 11 |
| SEQ ID No: 7 | SEQ ID NO: 12 |
| SEQ ID No: 8 | SEQ ID No: 13 |

Subsequently, following a second amplification reaction carried out according to the process described in Example 4, the DNA fragments from the first amplification reaction, corresponding to each of the herpesviruses (HSV1, HSV2, VZV, CMV, HHV6 and EBV), have been amplified. It has been possible to detect specific DNA of each of the herpesviruses and of PRV after the second amplification reaction using the mixture of specific primers constituted by:

| POSITIVE POLARITY | NEGATIVE POLARITY |
|---|---|
| SEQ ID No: 29 | SEQ ID No: 30 |
| SEQ ID No: 16 | SEQ ID No: 21 |
| SEQ ID No: 17 | SEQ ID No: 22 |
| SEQ ID No: 18 | SEQ ID No: 23 |
| SEQ ID No: 19 | SEQ ID No: 24 |
| SEQ ID No: 20 | SEQ ID No: 25 |

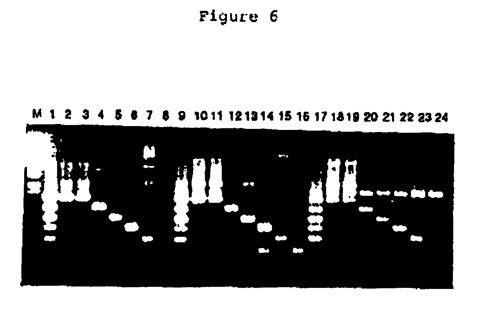

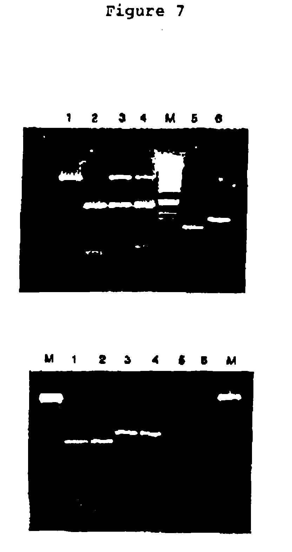

Each virus is identified as a function of the molecular weight of the amplified fragments Figure 6.

### Example 8. Detection of enteroviruses and herpesviruses in the presence of specific primers of the PRV virus used as internal control of the first and second amplification.

The internal amplification control designed in Example 6 has been used. The mixture of primers which is constituted by those specific to enteroviruses and to herpesviruses is enriched by the presence of the primers of the internal control, which consist in a cloned fragment of the DNA polymerase gene of the PRV virus.

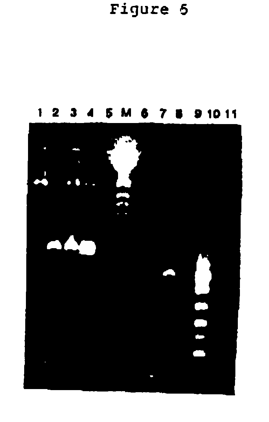

By using the primer mixtures for the general amplification of enteroviruses and herpesviruses designed in Example 1 and, reamplifying them under the conditions defined in example 4, 500 bp amplification fragments, specific of enteroviruses, and 140 bp fragments, specific of herpesviruses, have been obtained, when the first amplification reaction is carried out. In relation to the second amplification reaction, all the herpesviruses can be typed, and the presence of a 140 bp amplification fragment, which coincides with the fragments generated in Example 6, is demonstrated. The amplification fragments of the first and second reaction and amplification may be observed in Figure 5.

### Example 9. Design of the mixture of primers specific of HSV1 and HSV2.

There is a very high degree of homology between Herpes simplex virus type 1 and Herpes simplex virus type 2. In a manner similar to that in which the design of the primers of Example 6 was carried out, the nucleotide sequences of the specific primers of HSV1 and HSV2 have been determined, for use in a second amplification reaction in which the target molecule is the amplification fragment resulting from the first amplification reaction, previously developed and described in the Spanish Patent P9201174.

On the basis of the aligned and compared sequences of the human herpesviruses of Figure 2, specific primers have been designed for these two very homologous viruses.
- POSITIVE POLARITY:: SEQ ID No: 31
- NEGATIVE POLARITY:: SEQ ID No: 32

Amplification using the mixtures designed should yield fragments of 93 bp for HSV1 and of 138 for HSV2.

### Example 10. Detection of specific sequences of HSV1 or HSV2 in samples which may or may not contain enteroviral RNA.

Using the first amplification reaction and typification mixtures of primers of Examples 1 and 8, and the processes described in Examples 2 and 4, it has been possible to specifically identify amplification fragments of HSV1 or HSV2. As in the previous Examples 4 and 7, the presence of enteroviruses in the reaction mixture of the first amplification, as well as in that of the second amplification reaction, does not have any influence upon the characterization of the 93 bp and 138 bp specific amplification fragments, depending on them respectively being HSV1 or HSV2.

## Claims

1. A method for the differential diagnosis of infectious agents which are related due to their ability to produce a similar pathology, belonging to different families which are not genomically related to each other, which includes (a) the detection of said infectious agents by enzymatic amplification of a fragment of their genomes and (b) the identification of said infectious agents on the basis of the products of the amplification reaction, characterized in that said amplification reaction is carried out within a single reaction mixture which comprises, as primer for the reaction, a combination of oligonucleotide mixtures, each one of which has been specifically designed for each one of the families of infectious agents to be detected, so that each mixture of oligonucleotides:
i) is obtained by the simple addition of oligonucleotides,
ii) each one of the oligonucleotides comprised in said mixture include, preferably at their 3' end, homologous sequences selected from among the genomically related sequences which are desired to amplify,
iii) each one of the oligonucleotides comprised in said mixture may further include, preferably at their 5' end, non-homologous sequences selected from among the genomically related sequences which are desired to amplify, and
iv) the oligonucleotide components of which may be differentiated from the known sequences which are desired to amplify in at least one nucleotide.

2. A method according to claim 1, in which the amplification reaction mixture further includes an internal amplification control which comprises the genome of a virus which must not be present in the sample to be studied, together with reaction primers specific to a fragment of said genome.

3. A method according to claim 2, in which said genome is derived from a viral species belonging to one of the viral families to be identified.

4. A method according to claim 3, in which said genome corresponds to a virus which is incapable of infecting the animal species on which the diagnosis is carried out.

5. A method according to claim 4, in which said genome corresponds to a virus which is incapable of infecting human beings.

6. A method according to claim 5, in which said genome corresponds to a herpesvirus which is incapable of infecting man.

7. A method according to claim 6, in which said genome corresponds to Porcine herpesvirus 1.

8. A method according to claim 1, in which the identification of the infectious agent is carried out by hybridization of the amplified sequences with specific oligonucleotide probes or mixtures of specific oligonucleotides for the species or genera to be identified.

9. A method according to claim 1, in which the identification of the infectious agent is carried out by a second amplification reaction of the products resulting from the first amplification reaction, in which the reaction mixture for said second amplification reaction comprises, as primer for the reaction, a mixture of oligonucleotides designed in such a way that:
i) it is obtained by the simple addition of oligonucleotides,
ii) each one of the oligonucleotides comprised in said mixture include, preferably at their 3' end, specific sequences or each one of the sequences to be typified in said second amplification reaction,
iii) the specific amplified fragments differ from each other by reason of size or physical or chemical labelling of each one of the oligonucleotides comprised in said mixture, and
iv) one or more of the oligonucleotides comprised in said mixture may be differentiated from the known sequences which are desired to amplify in at least one nucleotide.

10. A method according to claim 1, in which said infectious agents produce a similar pathology in humans.

11. A method according to claim 1, in which said infectious agents belong to the *Herpesviridae* and *Picornaviridae* families.

12. A method according to claim 11, in which said infectious agents are human herpesviruses and enteroviruses.

13. A method according to claim 12, in which the amplification reaction mixture comprises:
- at least one oligonucleotide selected from among the group formed by the oligonucleotides identified as SEQ ID No: 1, SEQ ID No: 2 and SEQ ID No: 3; and
- at least one oligonucleotide selected from among the group formed by the oligonucleotides identified as SEQ ID No: 4, SEQ ID No:5, SEQ ID No: 6, SEQ ID No: 7, SEQ ID No: 8, SEQ ID No: 9, SEQ ID No: 10, SEQ ID No: 11, SEQ ID No: 12 and SEQ ID No: 13;
or their homologues in the complementary strands.

14. A method according to claim 13, in which the amplification reaction mixture also includes an internal amplification control which comprises a complete genome or a genomic fragment of Porcine herpesvirus 1, and reaction primers capable of amplifying a fragment thereof.

15. A method according to claim 14, in which said reaction primers capable of amplifying a fragment of the genome of Porcine herpesvirus 1 are selected from among the group formed by the oligonucleotides identified as SEQ ID No: 27, SEQ ID No: 29, SEQ ID No: 28 and SEQ ID No: 30, or their homologues in the complementary strands.

16. A method according to claim 12, in which the identification of the enteroviruses detected is carried out by specific hybridization with an oligonucleotide which comprises a sequence as the one identified as SEQ ID No: 26 or its homologue in the complementary strands.

17. A method according to claim 12, in which the identification of the herpesviruses detected is carried out by specific hybridization with a mixture of specific oligonucleotides specific for each one of the species or genera to be identified.

18. A method according to claim 17, in which after the identification of the herpesviruses detected, a specific hybridization reaction is carried out with an specific oligonucleotide for each species of herpesvirus to be identified.

19. A method according to claim 12, in which the identification of the viruses detected is carried out by means of a second amplification reaction, the reaction mixture of which comprises at least one of the oligonucleotides identified as SEQ ID No: 14, SEQ ID No: 16, SEQ ID No: 17, SEQ ID No: 18, SEQ ID No: 19, SEQ ID No: 20, SEQ ID No: 15, SEQ ID No: 21, SEQ ID No: 22, SEQ ID No: 23, SEQ ID No: 24 and SEQ ID No: 25, or their homologues in the complementary strands.

20. A method according to claim 19, in which after identifying the presence of the Herpes simplex virus (type 1 or type 2), a second genomic amplification reaction is carried out for its specific identification, the reaction mixture of which comprises at least one of the oligonucleotides identified as SEQ ID No: 31, SEQ ID No: 32, or their homologues in the complementary strands.

21. A combination of oligonucleotide mixtures, for use as primer in a genome amplification reaction for the detection of infectious agents which are related due to their ability to produce a similar pathology, belonging to different families which are not genomically related to each other, each one of these mixtures having been specifically designed for each of the families of infectious agents to be detected and also designed in such a way that each mixture of oligonucleotides:
i) is obtained by the simple addition of oligonucleotides,
ii) each one of the oligonucleotides comprised in said mixture include, preferably at their 3' end, homologous sequences selected from among the genomically related sequences which are desired to amplify,
iii) each one of the oligonucleotides comprised in said mixture may further include, preferably at their 5' end, non-homologous sequences selected from among the genomically related sequences which are desired to amplify, and
iv) the oligonucleotide components of which may be differentiated from the known sequences which are desired to amplify in at least one nucleotide.

22. A combination according to claim 21, useful as primer in a genome amplification reaction for the detection of infectious agents which are related due to their ability to produce a similar pathology in humans.

23. A combination according to claim 21, useful as primer in a genome amplification reaction for the detection of infectious agents which belong to the *Herpesviridae* and *Picornaviridae* families.

24. A combination according to claim 21, useful as primer in a genome amplification reaction for the detection of infectious agents selected from the group formed by human herpesviruses and enteroviruses.
